Europäisches Patentamt
European Patent Office
Office européen des brevets

(19)

(11) Publication number: **0 555 488 A1**

(12) # EUROPEAN PATENT APPLICATION published in accordance with Art. 158(3) EPC

(21) Application number: **92916529.8**

(22) Date of filing: **30.07.92**

(86) International application number: **PCT/JP92/00965**

(87) International publication number: **WO 93/03017 (18.02.93 93/05)**

(51) Int. Cl.5: **C07D 239/34**, C07D 239/47, C07D 239/48, C07D 239/52, C07D 239/60, C07D 251/14, C07D 251/42

(30) Priority: **31.07.91 JP 213085/91**

(43) Date of publication of application: **18.08.93 Bulletin 93/33**

(84) Designated Contracting States: **AT BE CH DE DK ES FR GB GR IT LI LU MC NL SE**

(71) Applicant: **KUMIAI CHEMICAL INDUSTRY CO., LTD.**
**4-26, Ikenohata 1-chome**
**Taitoh-ku Tokyo 110(JP)**
Applicant: **IHARA CHEMICAL INDUSTRY Co., Ltd.**
**4-26, Ikenohata 1-chome**
**Taitoh-ku, Tokyo 110(JP)**

(72) Inventor: **SATO, Masahiro, Kabushiki Kaisha K.I Kenkyusho**
**408-1, Shioshinden, Fukude-cho**
**Iwata-gun, Shizuoka 437-12(JP)**
Inventor: **KAKU, Koichiro, Kabushiki Kaisha K.I Kenkyusho**
**408-1, Shioshinden, Fukude-cho**
**Iwata-gun, Shizuoka 437-12(JP)**
Inventor: **TACHIKAWA, Shigehiko**
**57, Anzai 3-chome**
**Shizuoka-shi, Shizuoka 420(JP)**

(74) Representative: **Wächtershäuser, Günter, Dr.**
**Tal 29**
**D-80331 München (DE)**

(54) **PYRIMIDINE OR TRIAZINE DERIVATIVE AND HERBICIDE.**

(57) A herbicide containing a novel pyrimidine or triazine derivative represented by general formula (I) or a salt thereof as the active ingredient, wherein $R^1$ represents trifluoromethanesulfonyloxy etc.; $R^2$ represents hydrogen, lower alkyl, lower alkenyl, lower alkynyl, etc.; $R^3$ and $R^4$ may be the same or different from each other and each represents halogen, lower alkyl, lower alkoxy, difluoromethoxy, etc.; and Z represents nitrogen or methine. The above derivative has an excellent herbicidal effect on harmful weeds growing on lowland and upland fields and noncrop lands.

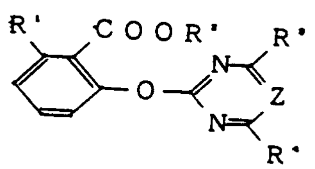
(I)

EP 0 555 488 A1

## TECHNICAL FIELD

The present invention relates to novel pyrimidine or triazine derivatives and their salts as well as herbicidal compositions containing said pyrimidine or triazine derivatives or their salts which can be applied to paddy fields, upland fields or non-agricultural fields.

## BACKGROUND ART

Heretofore, it is known that a compound having the formula,

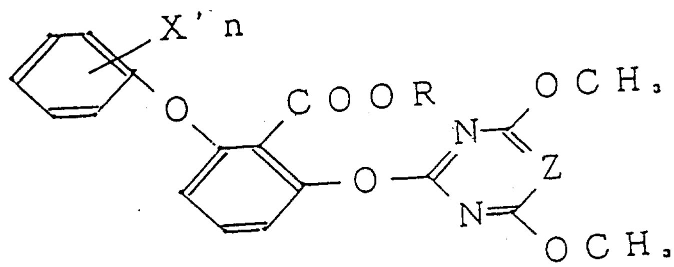

(wherein X' is a halogen atom, a lower alkyl group or lower alkoxy group, R is a hydrogen atom or a benzyl group, and n is an integer of 0, 1 or 2) has a herbicidal activity (Japanese Unexamined Patent Publication No. 250366/1989).

However, the herbicidal effect of the compound disclosed in the above patent publication is not always satisfactory, and a compound achieving a better herbicidal effect is demanded.

We have further studied about pyrimidine or triazine type compounds, aiming at developing a more improved compound. As a result of the study, the present inventors have found that a pyrimidine or triazine derivative having a trifluoromethanesulfonyloxy group, an ethenyl group which may optionally be substituted or an ethynyl group which may optionally be substituted on the benzene ring in place of a phenoxy group, achieves an excellent herbicidal effect on annual and perennial weeds in comparison with the compounds disclosed in the above cited Japanese publication, and have completed the present invention.

## DISCLOSURE OF THE INVENTION

The present invention relates to a pyrimidine or triazine derivative having the formula (1),

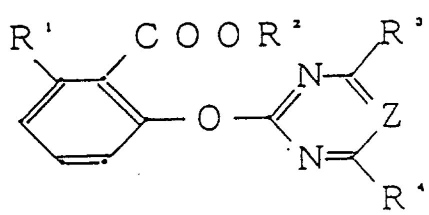

{wherein $R^1$ is a trifluoromethanesulfonyloxy group, a group of the formula,

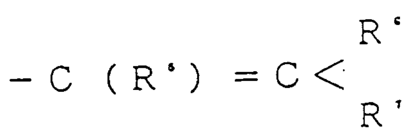

(wherein $R^5$ is a hydrogen atom, a chlorine atom, a lower alkyl group or a lower alkoxy group, $R^6$ is a hydrogen atom, a lower alkyl group, a cyano group or a nitro group, and $R^7$ is a hydrogen atom, a lower alkyl group, a phenyl group, an alkoxycarbonyl group, a haloalkoxycarbonyl group, a phenoxycarbonyl group, a cyano group, a nitro group, a thienyl group, a furyl group or a pyridyl group), a group of the formula -C≡C$R^8$ (wherein $R^8$ is a hydrogen atom, a lower alkyl group, a lower alkenyl group, a lower alkoxy

group, an alkoxyalkyl group, an alkylthioalkyl group, a phenyl group, a haloalkyl group, a cycloalkyl group, a cycloalkenyl group, a trimethylsilyl group, a cyanoalkyl group, a trifluoromethyl group, an acyloxyalkyl group, or an aminoalkyl group which may be substituted with an alkyl group or a benzyl group) or a group of the formula,

$$-(CH_2)_nN< \begin{matrix} R^{11} \\ R^{12} \end{matrix}$$

(wherein $R^{11}$ and $R^{12}$ are the same or different, and are a hydrogen atom, a lower alkyl group or a benzyl group, and n is 3 or 4);

$R^2$ is a hydrogen atom, a lower alkyl group, a lower alkenyl group, a lower alkynyl group, a benzyl group, a 2-trimethylsilylethyl group, an alkoxyalkyl group, an alkylthioalkyl group, an alkoxycarbonylalkyl group, a cyanoalkyl group, a nitroalkyl group, or a cation of an alkali metal, an alkali earth metal or an organic amine;

$R^3$ and $R^4$ are the same or different, and are a halogen atom, a lower alkyl group, a lower alkoxy group, a difluoromethoxy group, a trifluoromethyl group, or an amino group which may be substituted with a lower alkyl group;

X is an oxygen atom or a sulfur atom; and

Z is a nitrogen atom or a methine group}.

The present invention further relates to a herbicidal composition containing the above-mentioned pyrimidine or triazine derivative or its salt as an active ingredient.

Now, in the definition, the alkyl group of a lower alkyl group, an alkoxyalkyl group, an alkylthioalkyl group, a haloalkyl group, a cyanoalkyl group, a nitroalkyl group, an acyloxyalkyl group, or an aminoalkyl group which may be substituted with an alkyl group or a benzyl group, has a carbon number of from 1 to 6; the alkoxy group of a lower alkoxy group, an alkoxyalkyl group, an alkoxycarbonyl group, an alkoxycarbonylalkyl group, or a haloalkoxycarbonyl group, has a carbon number of from 1 to 6; the cycloalkyl group or the cycloalkenyl group has a carbon number of from 3 to 6; and the lower alkenyl group or the lower alkynyl group has a carbon number of from 2 to 6.

Also, in the definition of the formula (1), when illustrating preferable examples of substituents of $R^2$, the lower alkyl group is a methyl group, an ethyl group or a propyl group, the lower alkenyl group is an allyl group, the lower alkynyl group is a propargyl group, the alkoxyalkyl group is a methoxymethyl group, the alkylthioalkyl group is a methylthiomethyl group, the alkoxycarbonylalkyl group is a methoxycarbonylmethyl group or a 1-methoxycarbonylethyl group, the cyanoalkyl group is a cyanomethyl group, the nitroalkyl group is a nitromethyl group, the alkali metal is a sodium atom or a potassium atom, the alkaline earth metal is a calcium atom, and the organic amine cation is a diethylammonium cation or a diisopropylammonium cation.

Also, preferable examples of $R^3$ and $R^4$ include a chlorine atom as the halogen atom, a methyl group as the lower alkyl group, a methoxy group as the lower alkoxy group, and a dimethylamino group as the amino group which may be substituted with a lower alkyl group. Preferable examples of $R^5$ include a methyl group or an ethyl group as the lower alkyl group, and a methoxy group or an ethoxy group as the lower alkoxy group. Preferable examples of $R^6$ include a methyl group or an ethyl group as the lower alkyl group. Preferable examples of $R^7$ include a chlorine atom as the halogen atom, a methyl group or an ethyl group as the lower alkyl group, and a methoxycarbonyl group, a trifluoroethoxycarbonyl group or a phenoxycarbonyl group as the alkoxycarbonyl group. Preferable examples of $R^8$ include a methyl group, an ethyl group, a propyl group or a butyl group as the lower alkyl group, an allyl group as the lower alkenyl group, an ethoxy group as the lower alkoxy group, a methoxymethyl group as the alkoxyalkyl group, a methylthiomethyl group as the alkylthioalkyl group, a chloropropyl group as the haloalkyl group, a cyclopentyl group or a cyclohexyl group as the cycloalkyl group, a 1-cyclohexenyl group as the cycloalkenyl group, a cyanopropyl group as the cyanoalkyl group, and an acetoxymethyl group as the acyloxyalkyl group.

The compounds of the present invention can be produced in accordance with the following processes, but a production process is not limited to these processes.

Process A

As illustrated by the following reaction formula 1, a compound of the formula (1) of the present invention is produced by reacting a compound of the formula (2) with a pyrimidine or triazine compound of the

formula (3) in the presence of a base at a temperature preferably in the range of from room temperature to the boiling point of a solvent used.

Reaction formula 1

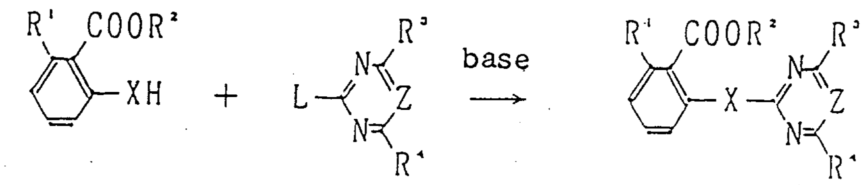


(2) (3) (1)

(wherein $R^1$, $R^2$, $R^3$, $R^4$, X and Z are as defined above,

Among the compounds of the formula (2), a compound of the formula (5) wherein $R^1$ is a trifluoromethanesulfonyloxy group and X is an oxygen atom can be produced by reacting a compound of the formula (4) with trifluoromethanesulfonic anhydride in the presence of a base preferably at a temperature in the range of from -20 °C to 5 °C.

Reaction formula 2

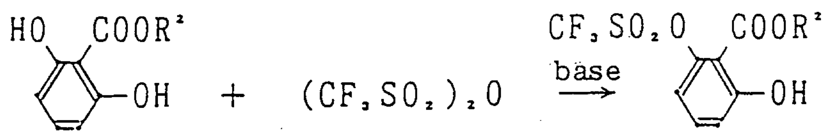


(4) (5)

(wherein $R^2$ is as defined above.)

The same base and the solvent as used in Process A can also be used in the above reaction, but preferable examples of the base include an organic amine such as pyridine, dimethylaminopyridine or triethylamine, a bicarbonate such as sodium hydrogencarbonate or potassium hydrogencarbonate, and a carbonate such as sodium carbonate or potassium carbonate. Preferable examples of the solvent include a halogenated hydrocarbon type solvent such as methylene chloride or chloroform, an ether type solvent such as ethyl ether or tetrahydrofuran, and an aprotic polar solvent such as N,N-dimethylformamide, N,N-dimethylacetamide or dimethylsulfoxide.

Process A-2

Among the compounds of the formula (2), a compound of the formula (8) wherein $R^1$ is a group other than a trifluoromethanesulfonyloxy group and X is an oxygen atom, can be produced by reacting a compound of the formula (6) with a compound of the formula (7) in the presence of a base and a catalyst preferably at a temperature in the range of from room temperature to the boiling point of a solvent.

Reaction formula 3

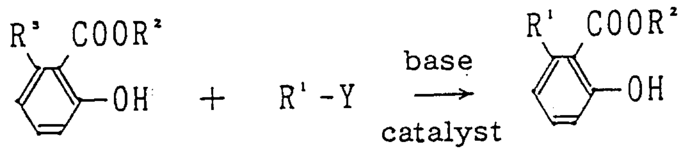


(6) (7) (8)

(wherein $R^1$ is a group represented by the formula $-C(R^5)=CR^6CR^7$ or $-C\equiv CR^8$, and $R^2$, $R^5$, $R^6$, $R^7$ and $R^8$ are as defined above. $R^9$ is a halogen atom or a trifluoromethanesulfonyloxy group, and Y is a hydrogen atom or a trialkyl tin group.)

Examples of the catalyst used herein, include tetrakis (triphenylphosphine)palladium(0), bis-(triphenylphosphine)palladium dichloride and the like, and other examples include a palladium catalyst such as (palladium acetate, triphenylphosphine) or [palladium acetate, tri(o-tolyl)phosphine]. Examples of the base used herein, include a carbonate such as sodium carbonate or potassium carbonate, a bicarbonate such as sodium hydrogencarbonate, and an organic amine such as triethylamine or pyridine. Examples of the solvent used herein, include a hydrocarbon type solvent such as benzene or toluene, an alcohol type solvent such as ethanol or methanol, an ether type solvent such as ethyl ether, tetrahydrofuran or 1,2-dimethoxyethane, an aprotic polar solvent such as N,N-dimethylformamide, N,N-dimethylacetamide or dimethylsulfoxide, an ester type solvent such as ethyl acetate, a ketone type solvent such as acetone or methyl ethyl ketone, acetonitrile, and others.

Process A-3

Among the compounds of the formula (2), a compound of the formula (11) wherein $R^1$ is a group of the formula $-CH=CR^6R^7$ and X is an oxygen atom, can be produced by reacting a carbonyl compound with a compound of the formula (9) in the presence of a base preferably at a temperature in the range of from -78°C to 50°C to prepare a compound of the formula (10) and further reacting the compound of the formula (10) thus prepared, with a demethylating agent preferably at a temperature in the range of from -78°C to room temperature.

Reaction formula 4

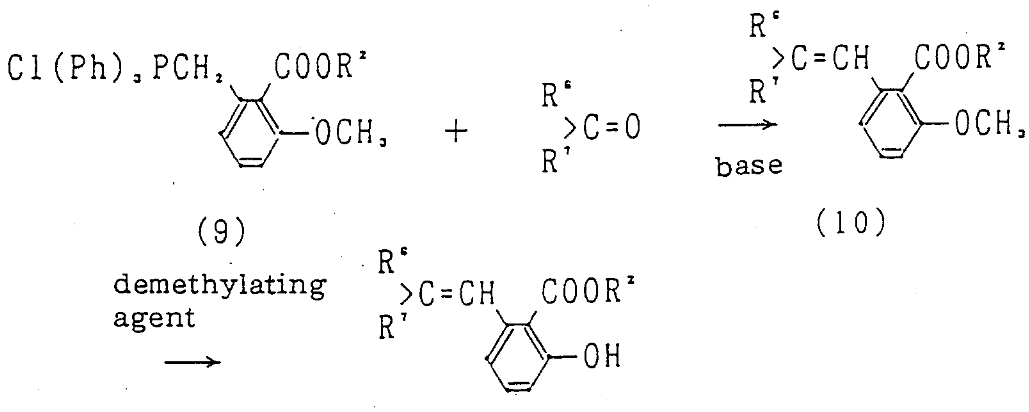


(9) (10)

(11)

Preferable examples of the base used in the above Wittig reaction, include a lithium reagent such as an alkyl lithium or a lithium bis(trialkylsilyl)amide, an alkali hydride such as sodium hydride, a carbonate such

as potassium carbonate, and an alkali metal alkoxide such as potassium tert-butoxide, but sodium amide or others can also be used. Also, the demethylation, (wherein $R^1$ and $R^2$ are as defined above.)

Process B

A compound of the formula (1) of the present invention can be produced by conducting the following reaction using a compound of the formula (14). The same catalyst, base and solvent as used in the above process A-2 can also be used herein.

Reaction formula 6

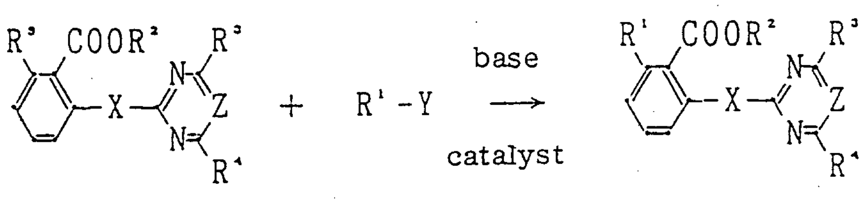


(14) (7) (1)

(wherein $R^1$ is a group represented by the formula

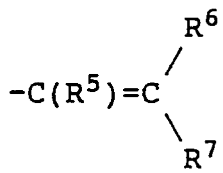

or the formula $-C \equiv CR^8$; $R^3$ and $R^4$ are the same or different and are a lower alkyl group, a lower alkoxy group, a difluoromethoxy group, a trifluoromethyl group or an amino group which may be substituted with a lower alkyl group; and $R^2$, $R^5$, $R^6$, $R^7$, $R^8$, $R^9$, X, Y and Z are as defined above.)

Process C

Among the compounds of the formula (1) of the present invention, a compound of the formula (16) wherein $R^2$ is a hydrogen atom, can be obtained by hydrolyzing its ester residue. Its process is not specified, but in the present invention, it was produced by reacting a fluorine reagent with a compound of the formula (15) wherein $R^2$ in the formula (1) is a 2-trimethylsilyl ethyl group, preferably at a temperature in the range of from room temperature to the boiling point of a solvent in the following manner.

Reaction formula 7

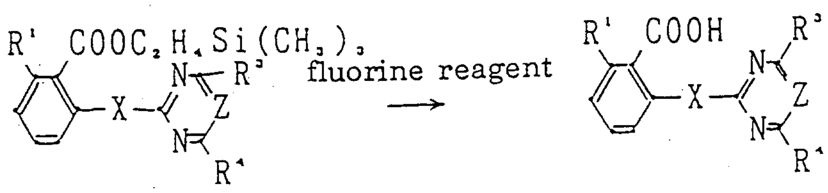


(15) (16)

(wherein X, Y, $R^1$, $R^3$ and $R^4$ are as defined above.)

Preferable examples of the fluorine reagent used herein, include a quaternary ammonium fluoride compound such as tributylammonium fluoride, and an alkali metal fluoride such as potassium fluoride or cesium fluoride. Preferable examples of the solvent used herein, include a hydrocarbon type solvent such as benzene or toluene, a halogenated hydrocarbon type solvent such as methylene chloride or chloroform, an alcohol type solvent such as ethanol or methanol, an ether type solvent such as ethyl ether, tetrahydrofuran or 1, 2-dimethoxyethane, an aprotic polar solvent such as N,N-dimethylformamide, N,N-dimethylacetamide or dimethylsulfoxide, an ester type solvent such as ethyl acetate or methyl acetate, and a ketone type solvent such as acetone or methyl ethyl ketone.

Process D

A compound of the formula (17) wherein $R^2$ in the formula (1) is a cation of an alkali metal, an alkaline earth metal or an organic amine, can be produced by reacting a compound of the formula (16) wherein $R^2$ in the formula (1) is a hydrogen atom, which is prepared for example by the above Process C, with a base preferably at a temperature in the range of from room temperature to the boiling point of a solvent.

Reaction formula 8

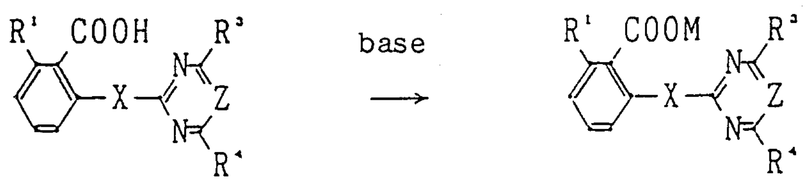


(16) (17)

(wherein $R^1$, $R^3$, $R^4$, X and Y are as defined above. M is a cation of an alkali metal such as sodium or potassium, an alkali earth metal such as calcium, or an organic amine.)

Examples of the base used herein, include an alkali metal such as metallic sodium or metallic potassium, a hydrogenated alkali metal or alkali earth metal such as sodium hydride, potassium hydride or calcium hydride, a carbonate such as sodium carbonate, potassium carbonate or calcium carbonate, and an alkali metal hydroxide such as sodium hydroxide or potassium hydroxide. Examples of the organic base include a monoalkylamine such as ammonia or methylamine, a dialkylamine such as dimethylamine, a trialkylamine such as triethylamine, and an aromatic amine such as pyridine.

Examples of the solvent used herein, include water, a hydrocarbon type solvent such as benzene or toluene, a halogenated hydrocarbon type solvent such as methylene chloride or chloroform, an alcohol type solvent such as ethanol or methanol, an ether type solvent such as ethyl ether, tetrahydrofuran, 1,2-dimethoxyethane or 1,4-dioxane, an ester type solvent such as ethyl acetate, a ketone type solvent such as acetone or methyl ethyl ketone, and acetonitrile.

Process E

A compound of the formula (19) wherein $R^2$ in the formula (1) is a lower alkyl group, a lower alkenyl group, a lower alkynyl group, an alkoxyalkyl group, a benzyl group, an alkylthioalkyl group, an alkoxycarbonylalkyl group, a cyanoalkyl group or a nitroalkyl group, can be produced by reacting a compound of the formula (18) with a compound of the formula (16) wherein $R^2$ in the formula (1) is a hydrogen atom, which is prepared for example by Process C, in the presence of an appropriate base preferably at a temperature in the range of from room temperature to the boiling point of a solvent.

Reaction formula 9

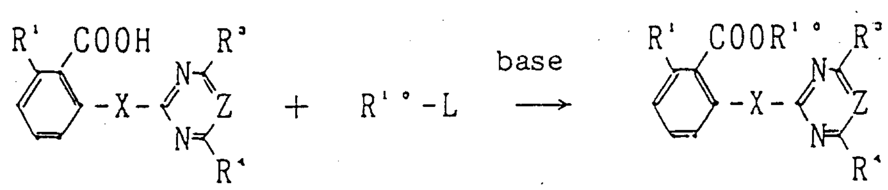


(16) (18) (19)

(wherein $R^1$, $R^3$, $R^4$, X, Y and L are as defined above, and $R^{10}$ is a lower alkyl group, a lower alkenyl group, a lower alkynyl group, an alkoxyalkyl group, a benzyl group, an alkylthioalkyl group, a cyanoalkyl group, a nitroalkyl group or an alkoxycarbonylalkyl group.)

Examples of the base used herein, include an alkali metal such as metallic sodium or metallic potassium, a hydrogenated alkali metal such as sodium hydride or potassium hydride, a carbonate such as sodium carbonate or potassium carbonate, a bicarbonate such as sodium hydrogencarbonate or potassium hydrogencarbonate, and an organic amine such as triethylamine, N,N-diisopropylethylamine or pyridine. Examples of the solvent used herein, include a hydrocarbon type solvent such as benzene or toluene, an ether type solvent such as ethyl ether, tetrahydrofuran, 1,2-dimethoxyethane or 1,4-dioxane, an ester type solvent such as ethyl acetate, a ketone type solvent such as acetone or methyl ethyl ketone, a halogen type solvent such as methylene chloride or chloroform, an aprotic polar solvent such as N,N-dimethylformamide, N,N-dimethylacetamide or dimethylsulfoxide, and acetonitrile.

Process F

Among the compounds of the formula (1), a compound of the formula (21) wherein in the formula (1), $R^1$ is an aminoalkyl group, $R^2$ is a hydrogen atom or a methyl group and X is an oxygen atom, can be produced by reducing a compound of the formula (20) wherein in the formula (1), $R^1$ is an aminoalkynyl group and $R^2$ is a benzyl group or a methyl group, with a hydrogen gas in the presence of an appropriate catalyst.

Reaction formula 10

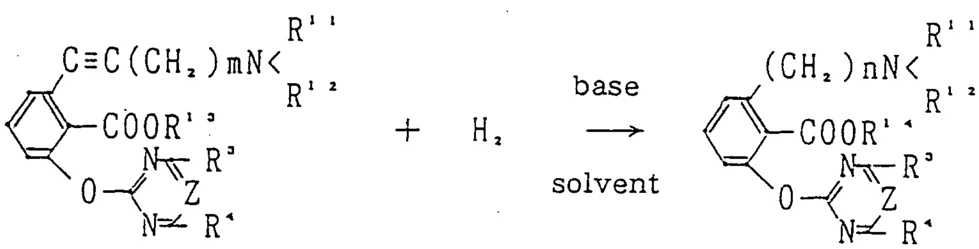


(20) (18) (21)

(wherein $R^{13}$ is a methyl group or a benzyl group; $R^{14}$ is a hydrogen atom or a methyl group; and $R^3$, $R^4$, $R^{11}$, $R^{12}$, Z, m and n are as defined above.)

Examples of the solvent used herein, include a hydrocarbon type solvent such as benzene or toluene, an alcohol type solvent such as methanol or ethanol, an ether type solvent such as ethyl ether, tetrahydrofuran, 1,2-dimethoxyethane or 1,4-dioxane, an ester type solvent such as ethyl acetate, a ketone type solvent such as acetone or methyl ethyl ketone, a halogen type solvent such as methylene chloride or chloroform, an aprotic polar solvent such as N,N-dimethylformamide, N,N-dimethylacetamide or dimethylsulfoxide, water, acetic acid, and formic acid.

Examples of the catalyst include Raney nickel and palladium carbon, but perchloric acid is added only when either of $R^{11}$ or $R^{12}$ contains a benzyl group and the benzyl group is eliminated.

The herbicide of the present invention comprises a pyrimidine or triazine derivative of the formula (1) as an active ingredient.

The compound of the present invention, as a herbicide, may be used as it is or may be formulated in various formulations such as a dust, a wettable powder, an emulsifiable concentrate a fine particle or a granule by blending it with a carrier, a surfactant, a dispersing agent or an adjuvant, which is commonly employed for the formulation of agricultural chemicals.

As the carrier to be used for the formulation, there may be enumerated a solid carrier such as zeeklite, talc, bentonite, clay, kaolin, diatomaceous earth, white carbon, vermiculite, calcium carbonate, slaked lime, silica sand, ammonium sulfate or urea, or a liquid carrier such as isopropyl alcohol, xylene, cyclohexanone, methylnaphthalene or isophorone.

As the surfactant and dispersing agent, there may be enumerated a metal salt of alkylbenzenesulfonic acid, a metal salt of dinaphthylmethanedisulfonic acid, an alcohol-sulfuric acid ester salt, an alkylarylsulfonate, a lignin sulfonate, a polyoxyethylene glycol ether, a polyoxyethylene alkylaryl ether or a polyoxyethylene sorbitol monoalkylate. As the adjuvant, there may be enumerated carboxymethyl cellulose, polyethylene glycol or gum arabic,

Table 1

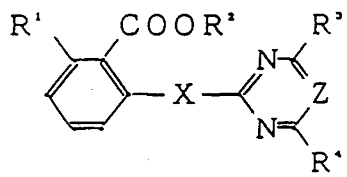

| Compound No. | $R^1$ | $R^2$ | $R^3$ | $R^4$ | X | Y | Melting point (°C) or Refractive index ($n^{20}$) |
|---|---|---|---|---|---|---|---|
| 1 | $CH=CHCH_3$ | $C_2H_4Si(CH_3)_3$ | $OCH_3$ | $OCH_3$ | O | CH | 1.5341 |
| 2 | $CH=CHCH_3$ | H | $OCH_3$ | $OCH_3$ | O | CH | 80~82 |
| 3 | $CH=CHCH_3$ | $CH_3$ | $OCH_3$ | $OCH_3$ | O | CH | 1.5578 |
| 4 | $CH=CHCH_3$ | $C_2H_5$ | $OCH_3$ | $OCH_3$ | O | CH | 65~68.5 |
| 5 | $CH=CHCH_3$ | $C_3H_7$ | $OCH_3$ | $OCH_3$ | O | CH | 1.5440 |
| 6 | $CH=CHCH_3$ | $C_3H_7$-i | $OCH_3$ | $OCH_3$ | O | CH | 1.5451 |
| 7 | $CH=CHCH_3$ | $CH_2OCH_3$ | $OCH_3$ | $OCH_3$ | O | CH | |
| 8 | $CH=CHCH_3$ | $CH_2CH=CH_2$ | $OCH_3$ | $OCH_3$ | O | CH | 1.5488 |
| 9 | $CH=CHCH_3$ | $CH_2C{\equiv}CH$ | $OCH_3$ | $OCH_3$ | O | CH | 108~110 |
| 10 | $CH=CHCH_3$ | $CH_2Ph$ | $OCH_3$ | $OCH_3$ | O | CH | 1.5699 |
| 11 | $CH=CHCH_3$ | $CH_2OC_2H_5$ | $OCH_3$ | $OCH_3$ | O | CH | 1.5469 |
| 12 | $CH=CHCH_3$ | $CH_2CO_2CH_3$ | $OCH_3$ | $OCH_3$ | O | CH | |
| 13 | $CH=CHCH_3$ | $CH<\begin{matrix}CO_2CH_3\\CH_3\end{matrix}$ | $OCH_3$ | $OCH_3$ | O | CH | 1.5456 |
| 14 | $CH=CHCH_3$ | $CH_2NO_2$ | $OCH_3$ | $OCH_3$ | O | CH | |
| 15 | $CH=CHCH_3$ | $CH_2CN$ | $OCH_3$ | $OCH_3$ | O | CH | 1.5544 |
| 16 | $CH=CHCH_3$ | $CH_2SCH_3$ | $OCH_3$ | $OCH_3$ | O | CH | 1.5638 |
| 17 | $CH=CHCH_3$ | Na | $OCH_3$ | $OCH_3$ | O | CH | 223~228 |
| 18 | $CH=CHCH_3$ | 1/2-Ca | $OCH_3$ | $OCH_3$ | O | CH | |
| 19 | $CH=CHCH_3$ | $H_2N<\begin{matrix}C_3H_7\text{-i}\\C_3H_7\text{-i}\end{matrix}$ | $OCH_3$ | $OCH_3$ | O | CH | 121~135 |
| 20 | $CH=CHCH_3$ | $C_2H_4Si(CH_3)_3$ | $OCH_3$ | $OCH_3$ | S | CH | |
| 21 | $CH=CHCH_3$ | H | $OCH_3$ | $OCH_3$ | S | CH | 145~147 |
| 22 | $CH=CHCH_3$ | $C_2H_4Si(CH_3)_3$ | Cl | $OCH_3$ | O | CH | |

| | | | | | | | |
|---|---|---|---|---|---|---|---|
| 23 | $CH=CHCH_3$ | H | Cl | $OCH_3$ | O | CH | |
| 24 | $CH=CHCH_3$ | $C_2H_4Si(CH_3)_3$ | $CF_3$ | $OCH_3$ | O | CH | 1.5029 |
| 25 | $CH=CHCH_3$ | H | $CF_3$ | $OCH_3$ | O | CH | 132~137 |
| 26 | $CH=CHCH_3$ | $C_2H_4Si(CH_3)_3$ | $N(CH_3)_2$ | $OCH_3$ | O | CH | |
| 27 | $CH=CHCH_3$ | H | $N(CH_3)_2$ | $OCH_3$ | O | CH | |
| 28 | $CH=CHCH_3$ | $C_2H_4Si(CH_3)_3$ | $CH_3$ | $CH_3$ | O | CH | 1.5342 |
| 29 | $CH=CHCH_3$ | H | $CH_3$ | $CH_3$ | O | CH | 135~139 |
| 30 | $CH=CHCH_3$ | $C_2H_4Si(CH_3)_3$ | $OCH_3$ | $OCH_3$ | O | N | 1.5295 |
| 31 | $CH=CHCH_3$ | H | $OCH_3$ | $OCH_3$ | O | N | 112~115 |
| 32 | $CH=CHCH_3$ | $CH_3$ | $OCH_3$ | $OCH_3$ | O | N | Unmeasurable |
| 33 | $CH=CHPh$ | $C_2H_4Si(CH_3)_3$ | $OCH_3$ | $OCH_3$ | O | CH | 1.5375 |
| 34 | $CH=CHPh$ | H | $OCH_3$ | $OCH_3$ | O | CH | 134~136 |
| 35 | $CH=CHCO_2CH_3$ | $C_2H_4Si(CH_3)_3$ | $OCH_3$ | $OCH_3$ | O | CH | 1.5392 |
| 36 | $CH=CHCO_2CH_3$ | H | $OCH_3$ | $OCH_3$ | O | CH | 95~98 |
| 37 | $C≡CSi(CH_3)_3$ | $C_2H_4Si(CH_3)_3$ | $OCH_3$ | $OCH_3$ | O | CH | 1.5285 |
| 38 | $C≡CH$ | $C_2H_4Si(CH_3)_3$ | $OCH_3$ | $OCH_3$ | O | CH | 1.5342 |
| 39 | $C≡CH$ | $H_2N<$ $C_3H_7$-i<br>$C_3H_7$-i | $OCH_3$ | $OCH_3$ | O | CH | |
| 40 | $OSO_2CF_3$ | $C_2H_4Si(CH_3)_3$ | $OCH_3$ | $OCH_3$ | O | CH | 1.4951 |
| 41 | $CH=CH_2$ | $C_2H_4Si(CH_3)_3$ | $OCH_3$ | $OCH_3$ | O | CH | 1.5310 |
| 42 | $CH=CH_2$ | H | $OCH_3$ | $OCH_3$ | O | CH | 112~117 |
| 43 | $C≡C-CH_2N<$ $CH_3$<br>$CH_3$ | $C_2H_4Si(CH_3)_3$ | $OCH_3$ | $OCH_3$ | O | CH | 1.5351 |
| 44 | $C≡C-CH_2N<$ $CH_3$<br>$CH_3$ | $H_2N<$ $C_3H_7$-i<br>$C_3H_7$-i | $OCH_3$ | $OCH_3$ | O | CH | |
| 45 | C≡C-Ph | $C_2H_4Si(CH_3)_3$ | $OCH_3$ | $OCH_3$ | O | CH | Unmeasurable |
| 46 | C≡C-Ph | $H_2N<C_2H_5)_2$ | $OCH_3$ | $OCH_3$ | O | CH | 107~116 |
| 47 | $C≡C-C_4H_9$ | $C_2H_4Si(CH_3)_3$ | $OCH_3$ | $OCH_3$ | O | CH | 1.5322 |
| 48 | $C≡C-C_4H_9$ | $H_2N<$ $C_3H_7$-i<br>$C_3H_7$-i | $OCH_3$ | $OCH_3$ | O | CH | |
| 49 | $C≡C-C_4H_9$-t | $C_2H_4Si(CH_3)_3$ | $OCH_3$ | $OCH_3$ | O | CH | 1.5312 |

| | | | | | | | |
|---|---|---|---|---|---|---|---|
| 72 | CH=CH-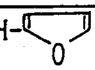 | $C_2H_4Si(CH_3)_3$ | $OCH_3$ | $OCH_3$ | 0 | CH | |
| 73 | CH=CH-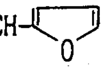 | H | $OCH_3$ | $OCH_3$ | 0 | CH | |
| 74 | CH=CH-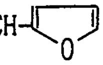 | $CH_3$ | $OCH_3$ | $OCH_3$ | 0 | CH | |
| 75 | CH=CH-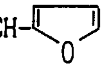 | $C_2H_4Si(CH_3)_3$ | $OCH_3$ | $OCH_3$ | 0 | CH | |
| 76 | CH=CH-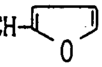 | H | $OCH_3$ | $OCH_3$ | 0 | CH | |
| 77 | CH=CH-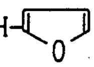 | $CH_3$ | $OCH_3$ | $OCH_3$ | 0 | CH | |
| 78 | $CH=C<^{CH_3}_{CH_3}$ | $C_2H_4Si(CH_3)_3$ | $OCH_3$ | $OCH_3$ | 0 | CH | 1.5232 |
| 79 | $CH=C<^{CH_3}_{CH_3}$ | H | $OCH_3$ | $OCH_3$ | 0 | CH | Unmeasurable |
| 80 | $CH=C<^{CH_3}_{CH_3}$ | $CH_3$ | $OCH_3$ | $OCH_3$ | 0 | CH | |
| 81 | $CH=C<^{COOCH_3}_{CN}$ | $C_2H_4Si(CH_3)_3$ | $OCH_3$ | $OCH_3$ | 0 | CH | |
| 82 | $CH=C<^{COOCH_3}_{CN}$ | H | $OCH_3$ | $OCH_3$ | 0 | CH | |
| 83 | CH=CHCN(E) | $C_2H_4Si(CH_3)_3$ | $OCH_3$ | $OCH_3$ | 0 | CH | 1.5430 |
| 84 | CH=CHCN(E) | H | $OCH_3$ | $OCH_3$ | 0 | CH | 129~133 |
| 85 | CH=CHCN(Z) | $C_2H_4Si(CH_3)_3$ | $OCH_3$ | $OCH_3$ | 0 | CH | 1.5329 |
| 86 | CH=CHCN(Z) | H | $OCH_3$ | $OCH_3$ | 0 | CH | 165~170 |
| 87 | $CH=C<^{COOCH_3}_{NO_2}$ | $C_2H_4Si(CH_3)_3$ | $OCH_3$ | $OCH_3$ | 0 | CH | |
| 88 | $CH=C<^{COOCH_3}_{NO_2}$ | H | $OCH_3$ | $OCH_3$ | 0 | CH | |
| 89 | $CH=CHNO_2$ | $C_2H_4Si(CH_3)_3$ | $OCH_3$ | $OCH_3$ | 0 | CH | |
| 90 | $CH=CHNO_2$ | H | $OCH_3$ | $OCH_3$ | 0 | CH | |
| 91 | $CCl=CH_2$ | $C_2H_4Si(CH_3)_3$ | $OCH_3$ | $OCH_3$ | 0 | CH | |
| 92 | $CCl=CH_2$ | H | $OCH_3$ | $OCH_3$ | 0 | CH | |
| 93 | C≡C-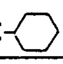 | $C_2H_4Si(CH_3)_3$ | $OCH_3$ | $OCH_3$ | 0 | CH | 1.5485 |

| | | | | | | | |
|---|---|---|---|---|---|---|---|
| 94 | $C\equiv C-$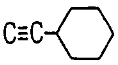 | $H_2N<^{C_3H_7-i}_{C_3H_7-i}$ | $OCH_3$ | $OCH_3$ | 0 | CH | 82~103 |
| 95 | $C\equiv C-$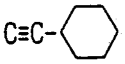 | $CH_3$ | $OCH_3$ | $OCH_3$ | 0 | CH | |
| 96 | $C\equiv C-OC_2H_5$ | $C_2H_4Si(CH_3)_3$ | $OCH_3$ | $OCH_3$ | 0 | CH | |
| 97 | $C\equiv C-OC_2H_5$ | $H_2N<^{C_3H_7-i}_{C_3H_7-i}$ | $OCH_3$ | $OCH_3$ | 0 | CH | |
| 98 | $C\equiv C-OC_2H_5$ | $CH_3$ | $OCH_3$ | $OCH_3$ | 0 | CH | |
| 99 | $C\equiv C-$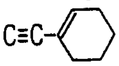 | $C_2H_4Si(CH_3)_3$ | $OCH_3$ | $OCH_3$ | 0 | CH | |
| 100 | $C\equiv C-$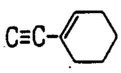 | $H_2N<^{C_3H_7-i}_{C_3H_7-i}$ | $OCH_3$ | $OCH_3$ | 0 | CH | |
| 101 | $C\equiv C-$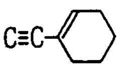 | $CH_3$ | $OCH_3$ | $OCH_3$ | 0 | CH | |
| 102 | $C\equiv C-CH_2CH_2CH_2CN$ | $C_2H_4Si(CH_3)_3$ | $OCH_3$ | $OCH_3$ | 0 | CH | 1.5360 |
| 103 | $C\equiv C-CH_2CH_2CH_2CN$ | $H_2N<^{C_3H_7-i}_{C_3H_7-i}$ | $OCH_3$ | $OCH_3$ | 0 | CH | 130~136 |
| 104 | $C\equiv C-CH_2CH_2CH_2CN$ | $CH_3$ | $OCH_3$ | $OCH_3$ | 0 | CH | Unmeasurable |
| 105 | $C\equiv C-CH_2OCH_3$ | $C_2H_4Si(CH_3)_3$ | $OCH_3$ | $OCH_3$ | 0 | CH | |
| 106 | $C\equiv C-CH_2OCH_3$ | $H_2N<^{C_3H_7-i}_{C_3H_7-i}$ | $OCH_3$ | $OCH_3$ | 0 | CH | |
| 107 | $C\equiv C-CH_2OCH_3$ | $CH_3$ | $OCH_3$ | $OCH_3$ | 0 | CH | |
| 108 | $C\equiv C-\underset{CH_3}{\underset{\mid}{C}}=CH_2$ | $C_2H_4Si(CH_3)_3$ | $OCH_3$ | $OCH_3$ | 0 | CH | |
| 109 | $C\equiv C-C(CH_3)=CH_2$ | $H_2N<^{C_3H_7-i}_{C_3H_7-i}$ | $OCH_3$ | $OCH_3$ | 0 | CH | |
| 110 | $C\equiv C-\underset{CH_3}{\underset{\mid}{C}}=CH_2$ | $CH_3$ | $OCH_3$ | $OCH_3$ | 0 | CH | |
| 111 | $C\equiv C-$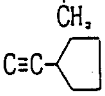 | $C_2H_4Si(CH_3)_3$ | $OCH_3$ | $OCH_3$ | 0 | CH | Unmeasurable |
| 112 | $C\equiv C-$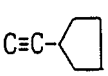 | $H_2N<^{C_3H_7-i}_{C_3H_7-i}$ | $OCH_3$ | $OCH_3$ | 0 | CH | 131~137 |
| 113 | $C\equiv C-$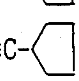 | $CH_3$ | $OCH_3$ | $OCH_3$ | 0 | CH | |
| 114 | $C\equiv C-CH_2SCH_3$ | $C_2H_4Si(CH_3)_3$ | $OCH_3$ | $OCH_3$ | 0 | CH | |
| 115 | $C\equiv C-CH_2SCH_3$ | $H_2N<^{C_3H_7-i}_{C_3H_7-i}$ | $OCH_3$ | $OCH_3$ | 0 | CH | |
| 116 | $C\equiv C-CH_2SCH_3$ | $CH_3$ | $OCH_3$ | $OCH_3$ | 0 | CH | |

| | | | | | | | |
|---|---|---|---|---|---|---|---|
| 117 | $C≡C-CF_3$ | $C_2H_4Si(CH_3)_3$ | $OCH_3$ | $OCH_3$ | O | CH | |
| 118 | $C≡C-CF_3$ | $C_3H_7$-i<br>$H_2N<$<br>$C_3H_7$-i | $OCH_3$ | $OCH_3$ | O | CH | |
| 119 | $C≡C-CF_3$ | $CH_3$ | $OCH_3$ | $OCH_3$ | O | CH | |
| 120 | $C≡C-CH_2OCOCH_3$ | $C_2H_4Si(CH_3)_3$ | $OCH_3$ | $OCH_3$ | O | CH | |
| 121 | $C≡C-CH_2OCOCH_3$ | $C_3H_7$-i<br>$H_2N<$<br>$C_3H_7$-i | $OCH_3$ | $OCH_3$ | O | CH | |
| 122 | $C≡C-CH_2OCOCH_3$ | $CH_3$ | $OCH_3$ | $OCH_3$ | O | CH | |
| 123 | $C≡C-CH_2CH=CH_2$ | $C_2H_4Si(CH_3)_3$ | $OCH_3$ | $OCH_3$ | O | CH | |
| 124 | $C≡C-CH_2CH=CH_2$ | $C_3H_7$-i<br>$H_2N<$<br>$C_3H_7$-i | $OCH_3$ | $OCH_3$ | O | CH | |
| 125 | $C≡C-CH_2CH=CH_2$ | $CH_3$ | $OCH_3$ | $OCH_3$ | O | CH | |
| 126 | $CH=CHCO_2CH_2CF_3$ | $C_2H_4Si(CH_3)_3$ | $OCH_3$ | $OCH_3$ | O | CH | 1.4925 |
| 127 | $CH=CHCO_2CH_2CF_3$ | H | $OCH_3$ | $OCH_3$ | O | CH | |
| 128 | $CH=CHCO_2C_6H_5$ | $C_2H_4Si(CH_3)_3$ | $OCH_3$ | $OCH_3$ | O | CH | |
| 129 | $CH=CHCO_2C_6H_5$ | H | $OCH_3$ | $OCH_3$ | O | CH | |
| 130 | $C=CHCO_2CH_3$<br>$CH_3$ | $C_2H_4Si(CH_3)_3$ | $OCH_3$ | $OCH_3$ | O | CH | |
| 131 | $C=CHCO_2CH_3$<br>$CH_3$ | H | $OCH_3$ | $OCH_3$ | O | CH | |
| 132 | $COOC_4H_9-t$<br>$CH=C<$<br>$CH_3$ | $C_2H_4Si(CH_3)_3$ | $OCH_3$ | $OCH_3$ | O | CH | 1.5132 |
| 133 | $COOC_4H_9-t$<br>$CH=C<$<br>$CH_3$ | H | $OCH_3$ | $OCH_3$ | O | CH | Unmeasurable |
| 134 | $COOC_4H_9-t$<br>$CH=C<$<br>$CH_3$ | $CH_2OCH_3$ | $OCH_3$ | $OCH_3$ | O | CH | 1.5223 |
| 135 | $COOCH_3$<br>$CH=C<$<br>$CH_3$ | H | $OCH_3$ | $OCH_3$ | O | CH | Unmeasurable |
| 136 | $CH=CHCH_3$ | $C_2H_4Si(CH_3)_3$ | $OCHF_2$ | $OCH_3$ | O | CH | 1.5215 |
| 137 | $CH=CHCH_3$ | H | $OCHF_2$ | $OCH_3$ | O | CH | 1.5189 |
| 138 | CH=CHCOOH | H | $OCH_3$ | $OCH_3$ | O | CH | 154-160 |
| 139 | $CH=CHCH_2CH_3$ | H | OCH | OCH | O | CH | 1.5591 |
| 140 | $OSO_2CF_3$ | $CH_2Ph$ | $OCH_3$ | $OCH_3$ | O | CH | 1.5319 |

| | | | | | | | |
|---|---|---|---|---|---|---|---|
| 141 | $OSO_2CF_3$ | H | $OCH_3$ | $OCH_3$ | O | CH | 1.34-136 |
| 142 | $C\equiv C-CH_2N{<}^{CH_3}_{CH_3}$ | $CH_2Ph$ | $OCH_3$ | $OCH_3$ | O | CH | Unmeasurable |
| 143 | $CH_2CH_2CH_2N{<}^{CH_3}_{CH_3}$ | H | $OCH_3$ | $OCH_3$ | O | CH | 205-208 |
| 144 | $CH_2CH_2CH_2N{<}^{CH_3}_{CH_3}$ | $CH_3$ | $OCH_3$ | $OCH_3$ | O | CH | |
| 145 | $C\equiv C-CH_2N{<}^{C_2H_5}_{C_2H_5}$ | $CH_2Ph$ | $OCH_3$ | $OCH_3$ | O | CH | |
| 146 | $CH_2CH_2CH_2N{<}^{C_2H_5}_{C_2H_5}$ | H | $OCH_3$ | $OCH_3$ | O | CH | |
| 147 | $CH_2CH_2CH_2N{<}^{C_2H_5}_{C_2H_5}$ | $CH_3$ | $OCH_3$ | $OCH_3$ | O | CH | |
| 148 | $C\equiv C-CH_2N{<}^{CH_3}_{CH_2Ph}$ | $CH_2Ph$ | $OCH_3$ | $OCH_3$ | O | CH | |
| 149 | $CH_2CH_2CH_2N{<}^{CH_3}_{CH_2Ph}$ | H | $OCH_3$ | $OCH_3$ | O | CH | |
| 150 | $CH_2CH_2CH_2N{<}^{CH_3}_{CH_2Ph}$ | $CH_3$ | $OCH_3$ | $OCH_3$ | O | CH | |
| 151 | $CH_2CH_2CH_2NHCH_2Ph$ | H | $OCH_3$ | $OCH_3$ | O | CH | |
| 152 | $\underset{OCH_3}{\underset{\mid}{C}}=CH_2$ | $CH_2Ph$ | $OCH_3$ | $OCH_3$ | O | CH | |
| 153 | $\underset{OCH_3}{\underset{\mid}{C}}=CH_2$ | H | $OCH_3$ | $OCH_3$ | O | CH | |

Now, the present invention will be described in further detail with reference to Examples. However, it should be understood that the present invention is by no means restricted to such specific Examples.

EXAMPLE 1

Preparation of 2-trimethylsilylethyl 2-[(4,6-dimethoxypyrimidin-2-yl)oxy]-6-(1-propenyl)benzoate (Compound No. 1)

In a 50 mℓ round bottom-flask, there were charged 2-trimethylsilylethyl 6-(1-propenyl)salicylate (3.1 g, 11.0 mmol), 4,6-dimethoxy-2-methanesulfonylpyrimidine (2.4 g, 11.0 mmol), potassium carbonate (1.8 g, 13.0 mmol) and 10 ml of N,N-dimethylformamide (hereinafter referred to as "DMF"), and the resultant mixture was reacted for 1 hour at 80°C. After cooling the reaction mixture, DMF was distilled off under reduced pressure, and the residue thus obtained was dissolved in ethyl acetate, washed with water and a saturated salt water, dried with anhydrous sodium sulfate and concentrated. The resultant residue was

purified by silica gel column chromatography to obtain 2.0 g (yield: 47.6%) of the aimed product (colorless transparent viscous liquid).

EXAMPLE 2

Preparation of 2-[(4,6-dimethoxypyrimidin-2-yl)oxy]-6-(1-propenyl)benzoic acid (Compound No. 2)

(2-Trimethylsilyl)ethyl 2-[(4,6-dimethoxypyrimidin-2-yl)oxy]-6-(1-propenyl)benzoate (2.0 g, 5.0 mmol) was dissolved in tetrahydrofuran (20 ml, hereinafter referred to as "THF") in a 50 ml round bottom-flask, and tetrabutylammonium fluoride hydrate (3.5 g, 13.3 mmol) was added thereto little by little. The resultant mixture was stirred at room temperature for 2 hours, and THF was distilled off under reduced pressure. To the residue thus obtained, was added water, and the resultant mixture was adjusted to pH 3 with 10% hydrochloric acid and was then extracted with ethyl acetate. The resultant product was then washed with water and a saturated salt water, dried with anhydrous sodium sulfate and concentrated. The residue thus obtained was washed with hexane, and the solvent was completely distilled off to obtain 1.0 g (yield: 66.7%) of the aimed product (red-brown glass-like material).

EXAMPLE 3

Preparation of 2-trimethylsilylethyl 2-[(4,6-dimethoxypyrimidin-2-yl)oxy]-6-vinylbenzoate (Compound No. 41)

In a 100 ml flask equipped with a thermometer, a cooling tube and a nitrogen-introducing tube, there were charged 2-trimethylsilylethyl 2-[(4,6-dimethoxypyrimidin-2-yl)oxy]-6-trifluoromethanesulfonyloxybenzoate (3.15 g, 6.0 mmol), tetrakis(triphenylphosphine)palladium(0) (400 mg, 0.34 mmol) and toluene (30 ml), and the resultant mixture was stirred at room temperature for 1 hour under nitrogen stream. After adding 2,6-di-tert-butyl-4-methylphenol (catalytic amount) to the resultant mixture, vinyl tributyl tin (3.17 g, 10.0 mmol) was further added and the mixture was reacted at 80-90 °C for 3 hours. After cooling the mixture, toluene was added thereto and the organic layer was washed with water and a saturated salt water, dried with anhydrous sodium sulfate and concentrated. The residue thus obtained was purified by silica gel column chromatography to obtain 0.50 g (yield: 20.8%) of the aimed product (transparent viscous orange color liquid).

EXAMPLE 4

Preparation of 2-trimethylsilylethyl 2-[(4,6-dimethoxypyrimidin-2-yl)oxy]-6-(1-pentynyl)benzoate (Compound No. 52)

In a 100 ml flask equipped with a thermometer, a cooling tube and a nitrogen-introducing tube, there were charged 2-trimethylsilylethyl 2-[(4,6-dimethoxypyrimidin-2-yl)oxy]-6-trifluoromethanesulfonyloxybenzoate (4.72 g, 9.0 mmol), bis(triphenylphosphine)palladium dichloride (300 mg, 0.4 mmol), DMF (30 ml) and triethylamine (5 ml), and the resultant mixture was stirred at room temperature for 1 hour under nitrogen stream. Thereafter, 1-pentyne (2.0 g, 29.40 mmol) was added to the resultant mixture, and the mixture was reacted at 80-90 °C for 3 hours. After cooling, the mixture was poured into an ice water, and was extracted with ethyl acetate. The organic layer thus obtained was washed with water and a saturated salt water, dried with anhydrous sodium sulfate and concentrated. The residue thus obtained was then purified by silica gel column chromatography to obtain 2.50 g (yield: 62.8%) of the aimed product (red-brown transparent viscous liquid).

EXAMPLE 5

Preparation of diisopropylammonium 2-[(4,6-dimethoxypyrimidin-2-yl)oxy]-6-(1-pentynyl)benzoate (Compound No. 53)

In a 100 ml round bottom-flask, there was charged 2-trimethylsilylethyl 2-[(4,6-dimethoxypyrimidin-2-yl)-oxy]-6-(1-pentynyl)benzoate (2.10 g, 4.7 mmol) dissolved in THF (50 ml), and tetrabutylammonium fluoride hydrate (1 mol-THF solution, 14.2 ml, 14.2 mmol) was added thereto. The resultant mixture was stirred at room temperature for 3 hours. After distilling off THF under reduced pressure, water was added thereto, and

the mixture was adjusted to pH 3 with 10% hydrochloric acid and was extracted with ethyl acetate. The organic layer thus obtained was washed with water and a saturated salt water, and dried with anhydrous sodium sulfate and concentrated. The resultant residue thus obtained was added to methanol (50 ml) having diisopropylamine (1.01 g, 10.0 mmol) dissolved, and the resultant mixture was stirred at 50 °C for 2 hours. After cooling, the solvent was distilled off, and the resultant product was recrystallized with isopropylether to obtain 1.74 g (yield: 83.7%) of the aimed product (light yellowish granular crystal).

EXAMPLE 6

Preparation of methyl 2-[(4,6-dimethoxypyrimidin-2-yl)oxy]-6-(1-pentynyl)benzoate (Compound No. 54)

Diisopropylammonium 2-[(4,6-dimethoxypyrimidin-2-yl)oxy]-6-(1-pentynyl)benzoate (1.00 g, 2.3 mmol) was dissolved in tetrahydrofuran (20 ml)-DMF (10 ml) in a 50 ml round bottom-flask, and methyl iodide (1.4 g, 10.0 mmol) was added thereto little by little. The resultant mixture was stirred at 50 °C for 2 hours and THF-DMF was distilled off under reduced pressure. Water was added to the resultant residue, and the residue was extracted with ethyl acetate. The organic layer thus obtained was washed with water and a saturated salt water, dried with anhydrous sodium sulfate and concentrated. To the resultant mixture, was added an appropriate amount of Florisil, and the mixture was eluted with ispropyl ether. The solvent was then distilled off under reduced pressure to obtain 0.61 g (yield: 76.3 %) of the aimed product (yellow viscous liquid).

EXAMPLE 7

Preparation of methyl 2-[(4,6-dimethoxypyrimidin-2-yl)-oxy-6-(3-dimethylaminopropyl)benzoate (Compound No. 144)

In a 100 ml round bottom-flask, there were charged benzyl 2-[(4,6-dimethoxypyrimidin-2-yl)oxy]-6-(3-dimethylamino-1-propynyl)benzoate (1.60 g, 3.6 mmol), 10% palladium carbon (0.2 g) and methanol (50 ml), and hydrogen gas was introduced therein. After confirming that a required amount of hydrogen was consumed, palladium carbon was filtrated out, and methanol was distilled off under reduced pressure. The residue thus obtained was purified by column chromatography using Florisil to obtain 0.50 g (yield: 38.8%) of the aimed product (yellow-brown powder).

Now, typical Formulation Examples for a herbicidal composition of the present invention will be given. However, it should be understood that the present invention is by no means restricted to these specific Formulation Examples, and the type of a compound, the type of an additive and the blending ratio may be widely varied. In these Examples, "part" means "part by weight".

FORMULATION EXAMPLE 1 (Wettable powder)

10 Parts of Compound No. 64, 0.5 part of polyoxyethylene octylphenyl ether, 0.5 part of a sodium salt of $\beta$-naphthalenesulfonic acid-formalin condensate, 20 parts of diatomaceous earth and 69 parts of clay were mixed and pulverized to obtain a wettable powder.

FORMULATION EXAMPLE 2 (Wettable powder)

10 Parts of Compound No. 58, 0.5 part of polyoxyethylene octyl, 0.5 part of a sodium salt of $\beta$-naphthalenesulfonic acid-formalin condensate, 20 parts of diatomaceous earth, 5 parts of white carbon and 64 parts of clay were mixed and pulverized to obtain a wettable powder.

FORMULATION EXAMPLE 3 (Wettable powder containing calcium carbonate)

10 Parts of Compound No. 18, 0.5 part of a sodium salt of $\beta$-naphthalenesulfonic acid-formalin condensate, 0.5 part of a mixture of sodium laurylsulfate and sodium sulfate, 20 parts of diatomaceous earth, 5 parts of white carbon and 64 parts of calcium carbonate were mixed and pulverized to obtain a wettable powder.

FORMULATION EXAMPLE 4 (Emulsifiable concentrate)

30 Parts of Compound No. 38, 60 parts of an equivalent mixture of xylene and isophorone, and 10 parts of a mixture of polyoxyethylene sorbitan alkylate, polyoxyethylene alkylaryl polymer and alkylaryl sulfonate, were fully mixed to obtain an emulsifiable concentrate.

FORMULATION EXAMPLE 5 (Granule)

3 Parts of Compound No. 2, 77 parts of a bulking agent comprising a 1/3 mixture of talc/bentonite, 5 parts of white carbon, 5 parts of a surfactant mixture of polyoxyethylene sorbitan alkylate, polyoxyethylene alkylaryl polymer and alkylaryl sulfonate, and 10 parts of water, were fully kneaded to obtain a paste-like material. The paste-like material was then extruded to a sieve aperture of 0.7 mm in diameter, and the extruded product was dried and cut into pieces of 0.5 to 1 mm in length to obtain granules.

Now, the effect of a compound of the present invention will be described with reference to Test Examples.

TEST EXAMPLE 1 (herbicidal effect test by paddy field soil treatment)

In a plastic pot filled with paddy field soil (surface area: 100 $cm^2$), seeds of barnyardgrass (Ec), monochoria (Mo) and bulrush (Sc) were sown after puddling and leveling, and water was flooded thereon to a depth of 3 cm. Next day, a wettable powder prepared in accordance with Formulation Example 1 was diluted with water, and was applied dropwise to the pot in an amount of 100 g/10 a as an active ingredient. Then, the pot was cultured in a green house, and evaluation was conducted on the 21st day after the treatment in accordance with the standard as identified in the following Table 2. The results are shown in the following Table 3.

Table 2

| Index No. | Herbicidal effects and phytotoxicity (degree of growth control) |
|---|---|
| 5 | Herbicidal effect: at least 90% control |
| 4 | Herbicidal effect: at least 70% and less than 90% |
| 3 | Herbicidal effect at least 50% and less than 70% |
| 2 | Herbicidal effect: at least 30% and less than 50% |
| 1 | Herbicidal effect: at least 10% and less than 30% |
| 0 | Herbicidal effect: at least 0% and less than 10% |

Table 3

| Compound No. | Herbicidal effect | | |
|---|---|---|---|
| | Ec | Mo | Sc |
| 2 | 5 | 5 | 5 |
| 11 | 5 | 5 | 5 |
| 13 | 5 | 5 | 5 |
| 21 | 5 | 5 | 5 |
| 34 | 5 | 5 | 5 |
| 36 | 4 | 5 | 5 |
| 37 | 5 | 5 | 5 |
| 38 | 5 | 5 | 5 |
| 42 | 5 | 5 | 5 |
| 46 | 5 | 5 | 5 |
| 50 | 5 | 5 | 5 |
| 53 | 5 | 5 | 5 |
| 54 | 5 | 5 | 5 |
| 55 | 5 | 5 | 5 |
| 58 | 5 | 5 | 5 |
| 59 | 5 | 5 | 5 |
| 60 | 5 | 5 | 5 |
| 63 | 5 | 5 | 5 |
| 64 | 5 | 5 | 5 |
| 79 | 5 | 5 | 5 |
| 112 | 5 | 5 | 5 |
| 137 | 5 | 5 | 5 |
| 139 | 5 | 5 | 5 |

TEST EXAMPLE 2 (herbicidal effect test by upland field soil treatment)

In a plastic pot filled with upland field soil (surface area: 120 $cm^2$), seeds of edible barnyardgrass (Ec), pale smartweed (Po), slender amaranth (Am), lambsquarters (Ch) and rice flatsedge (Ci) were sown and covered with soil. A predetermined amount of a wettable powder prepared in accordance with Formulation Example 1 was diluted with water and applied uniformly to the soil surface by a small-sized sprayer in an amount of 100 ℓ/10 a so that the dose of the active ingredient was 100 g/10 a. The pot was then cultured in a green house, and evaluation was conducted on the 21st day after the treatment in accordance with the standard as identified in the following Table 2. The test results are shown in the following Table 4.

Table 4

| Compound No. | Herbicidal effect | | | | |
|---|---|---|---|---|---|
| | Ec | Po | Am | Ch | Ci |
| 2 | 5 | 5 | 5 | 5 | 5 |
| 11 | 5 | 5 | 5 | 5 | 5 |
| 13 | 3 | 5 | 5 | 5 | 4 |
| 15 | 5 | 5 | 5 | 5 | 5 |
| 21 | 5 | 5 | 5 | 5 | 5 |
| 34 | 3 | 4 | 5 | 5 | 5 |
| 36 | 4 | 5 | 5 | 5 | 5 |
| 37 | 3 | 5 | 5 | 5 | 5 |
| 38 | 4 | 5 | 5 | 5 | 5 |
| 42 | 5 | 5 | 5 | 5 | 5 |
| 46 | 4 | 5 | 5 | 5 | 5 |
| 50 | 4 | 5 | 5 | 5 | 5 |
| 53 | 5 | 5 | 5 | 5 | 5 |
| 54 | 3 | 5 | 5 | 5 | 3 |
| 58 | 5 | 5 | 5 | 5 | 5 |
| 59 | 4 | 5 | 5 | 5 | 5 |
| 60 | 5 | 5 | 5 | 5 | 5 |
| 63 | 3 | 5 | 5 | 5 | 5 |
| 67 | 3 | 5 | 5 | 5 | 4 |
| 79 | 5 | 5 | 5 | 5 | 5 |
| 112 | 5 | 5 | 5 | 5 | 5 |
| 137 | 4 | 5 | 5 | 5 | 5 |
| 139 | 5 | 5 | 5 | 5 | 5 |
| 141 | 2 | 5 | 5 | 5 | 5 |

TEST EXAMPLE 3 (herbicidal effect test by upland field foliage treatment)

In a plastic pot filled with upland soil (surface area: 120 $cm^2$), seeds of edible barnyardgrass (Ec), pale smartweed (Po), slender amaranth (Am), lambsquarters (Ch) and rice flatsedge (Ci) were sown, and were cultured in a green house for 2 weeks. A predetermined amount of a wettable powder prepared in accordance with Formulation Example 1 was diluted with water, and applied by a small-sized sprayer onto the foliage in an amount of 100 ℓ/10 a so that the dose of the active ingredient was 100 g/10 a. The plants were cultured in a green house, and evaluation was conducted on the 14th day after the treatment in accordance with the standard as identified in the following Table 2. The test results are shown in the following Table 5.

Table 5

| Compound No. | Herbicidal effect | | | | |
|---|---|---|---|---|---|
| | Ec | Po | Am | Ch | Ci |
| 2 | 5 | 5 | 5 | 5 | 5 |
| 11 | 5 | 5 | 5 | 5 | 5 |
| 13 | 4 | 5 | 5 | 5 | 5 |
| 15 | 5 | 5 | 5 | 5 | 5 |
| 21 | 5 | 5 | 5 | 5 | 5 |
| 34 | 5 | 5 | 5 | 5 | 5 |
| 36 | 4 | 5 | 5 | 5 | 5 |
| 37 | 4 | 5 | 5 | 5 | 5 |
| 38 | 5 | 5 | 5 | 5 | 5 |
| 42 | 5 | 5 | 5 | 5 | 5 |
| 46 | 5 | 5 | 5 | 5 | 5 |
| 50 | 4 | 5 | 5 | 3 | 3 |
| 53 | 5 | 5 | 5 | 5 | 5 |
| 57 | 3 | 5 | 5 | 5 | 3 |
| 58 | 5 | 5 | 5 | 5 | 5 |
| 59 | 5 | 5 | 5 | 5 | 5 |
| 60 | 4 | 5 | 5 | 5 | 5 |
| 63 | 3 | 5 | 5 | 4 | 5 |
| 64 | 5 | 5 | 5 | 5 | 5 |
| 67 | 5 | 5 | 5 | 5 | 5 |
| 79 | 5 | 5 | 5 | 5 | 5 |
| 112 | 5 | 5 | 5 | 5 | 5 |
| 137 | 5 | 5 | 5 | 5 | 5 |
| 139 | 5 | 5 | 5 | 5 | 5 |
| 141 | 2 | 5 | 5 | 4 | 3 |

## Claims

**1.** A pyrimidine or triazine derivative having the formula,

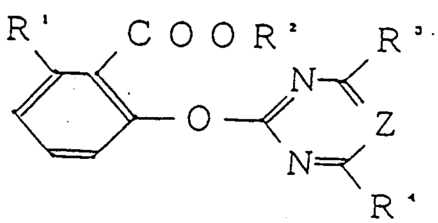

{wherein $R^1$ is (1) a trifluoromethanesulfonyloxy group,
(2) a group of the formula,

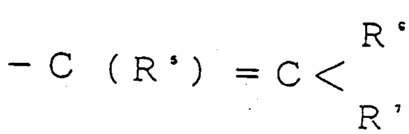

(wherein $R^5$ is a hydrogen atom, a chlorine atom, a lower alkyl group or a lower alkoxy group, $R^6$ is a hydrogen atom, a lower alkyl group, a cyano group or a nitro group, and $R^7$ is a hydrogen atom, a lower alkyl group, a phenyl group, an alkoxycarbonyl group, a haloalkoxycarbonyl group, a phenoxycarbonyl group, a cyano group, a nitro group, a thienyl group, a furyl group or a pyridyl group),
(3) a group of the formula $-C{\equiv}CR^8$ (wherein $R^8$ is a hydrogen atom, a lower alkyl group, a lower alkenyl group, a lower alkoxy group, an alkoxyalkyl group, an alkylthioalkyl group, a phenyl group, a haloalkyl group, a cycloalkyl group, a cycloalkenyl group, a trimethylsilyl group, a cyanoalkyl group, a trifluoromethyl group, an acyloxyalkyl group, or an aminoalkyl group which may be substituted with an alkyl group or a benzyl group), or
(4) a group of the formula,

$$-(CH_2)_nN<\begin{matrix}R^{11}\\R^{12}\end{matrix}$$

(wherein $R^{11}$ and $R^{12}$ are the same or different, and are a hydrogen atom, a lower alkyl group or a benzyl group, and n is 3 or 4);
$R^2$ is a hydrogen atom, a lower alkyl group, a lower alkenyl group, a lower alkynyl group, a benzyl group, a 2-trimethylsilylethyl group, an alkoxyalkyl group, an alkylthioalkyl group, an alkoxycarbonylalkyl group, a cyanoalkyl group, a nitroalkyl group, or a cation of an alkali metal, an alkali earth metal or an organic amine;
$R^3$ and $R^4$ are the same or different, and are a halogen atom, a lower alkyl group, a lower alkoxy group, a difluoromethoxy group, a trifluoromethyl group, or an amino group which may be substituted with a lower alkyl group;
X is an oxygen atom or a sulfur atom; and
Z is a nitrogen atom or a methine group}, or its salt.

2. The pyrimidine or triazine derivative or its salt according to Claim 1, wherein R' is a trifluoromethanesulfonyloxy group.

3. The pyrimidine or triazine derivative or its salt according to Claim 1, wherein R' is a group of the formula,

$$-C(R^5)=C<\begin{matrix}R^6\\R^7\end{matrix}$$

(wherein $R^5$ is a hydrogen atom, a chlorine atom, a lower alkyl group or a lower alkoxy group, $R^6$ is a hydrogen atom, a lower alkyl group, a cyano group or a nitro group, and $R^7$ is a hydrogen atom, a lower alkyl group, a phenyl group, an alkoxycarbonyl group, a haloalkoxycarbonyl group, a phenoxycarbonyl group, a cyano group, a nitro group, a thienyl group, a furyl group or a pyridyl group).

4. The pyrimidine or triazine derivative or its salt according to Claim 1, wherein R' is a group of the formula $-C{\equiv}CR^8$ (wherein $R^8$ is a hydrogen atom, a lower alkyl group, a lower alkenyl group, a lower alkoxy group, an alkoxyalkyl group, an alkylthioalkyl group, a phenyl group, a haloalkyl group, a cycloalkyl group, a cycloalkenyl group, a trimethylsilyl group, a cyanoalkyl group, a trifluoromethyl group, an acyloxyalkyl group, or an aminoalkyl group which may be substituted with an alkyl group or a benzyl group).

5. The pyrimidine or triazine derivative or its salt according to Claim 1, wherein R' is a group of the formula,

$$-(CH_2)_nN<\begin{matrix}R^{11}\\R^{12}\end{matrix}$$

(wherein $R^{11}$ and $R^{12}$ are the same or different, and are a hydrogen atom, a lower alkyl group or a benzyl group, and n is 3 or 4).

**6.** The pyrimidine or triazine derivative or its salt according to any of Claims 1 to 5, wherein $R^2$ is a methyl group, an ethyl group, a propyl group, an allyl group, a propargyl group, a methoxymethyl group, a methylthiomethyl group, a methoxycarbonylmethyl group, a 1-methoxycarbonylethyl group, a cyanomethyl group, a nitromethyl group, a sodium atom, a potassium atom, a calcium atom, a diethylammonium cation or a diisopropylammonium cation; $R^3$ and $R^4$ are a halogen atom, a methyl group, a methoxy group or a dimethylamino group; $R^5$ is a methyl group, an ethyl group, a methoxy group or an ethoxy group; $R^6$ is a methyl group or an ethyl group; $R^7$ is a halogen atom, a methyl group, an ethyl group, a methoxycarbonyl group, a trifluoroethoxycarbonyl group or a phenoxycarbonyl group; and $R^8$ is a methyl group, an ethyl group, a propyl group, a butyl group, an allyl group, an ethoxy group, a methoxymethyl group, a methylthiomethyl group, a chloropropyl group, a cyclopentyl group, a cyclohexyl group, a 1-cyclohexenyl group, a cyanopropyl group or an acetoxymethyl group.

**7.** The herbicidal composition containing the pyrimidine or triazine derivative or its salt as defined in any of Claims 1 to 6, as an active ingredient.

# INTERNATIONAL SEARCH REPORT

International Application No PCT/JP92/00965

**I. CLASSIFICATION OF SUBJECT MATTER** (if several classification symbols apply, indicate all)[6]

According to International Patent Classification (IPC) or to both National Classification and IPC

Int. $Cl^5$ C07D239/34, 239/47, 239/48, 239/52, 239/60, 251/14, 251/42

**II. FIELDS SEARCHED**

Minimum Documentation Searched[7]

| Classification System | Classification Symbols |
|---|---|
| IPC | C07D239/34-239/60, C07D251/14-251/42 |

Documentation Searched other than Minimum Documentation to the Extent that such Documents are Included in the Fields Searched[8]

| | |
|---|---|
| Jitsuyo Shinan Koho | 1926 - 1992 |
| Kokai Jitsuyo Shinan Koho | 1971 - 1992 |

**III. DOCUMENTS CONSIDERED TO BE RELEVANT**[9]

| Category[*] | Citation of Document,[11] with indication, where appropriate, of the relevant passages[12] | Relevant to Claim No.[13] |
|---|---|---|
| A | JP, A, 63-115870 (Kumiai Chemical Industry Co., Ltd.), May 20, 1988 (20. 05. 88), (Family: none) | 1-7 |
| A | JP, A, 62-270562 (ICI Australia Ltd.), November 24, 1987 (24. 11. 87), (Family: none) | 1-7 |
| A | JP, A, 62-174059 (Kumiai Chemical Industry Co., Ltd.), July 30, 1987 (30. 07. 87), (Family: none) | 1-7 |

* Special categories of cited documents:[10]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

**IV. CERTIFICATION**

| Date of the Actual Completion of the International Search | Date of Mailing of this International Search Report |
|---|---|
| October 13, 1992 (13. 10. 92) | November 2, 1992 (02. 11. 92) |
| **International Searching Authority** | **Signature of Authorized Officer** |
| Japanese Patent Office | |